# EUROPÄISCHE PATENTSCHRIFT

(11) **EP 1 615 663 B1**
(45) Veröffentlichungstag und Bekanntmachung des Hinweises auf die Patenterteilung: **26.12.2007**
(21) Anmeldenummer: 04790046.9
(22) Anmeldetag: 22.10.2004
(51) Int. Cl.: A61K 39/008

(54) **MITTEL ZUR BEHANDLUNG VON INFEKTIONEN MIT LEISHMANIEN**
AGENT FOR TREATING LEISHMANIA INFECTIONS
PRODUIT DE TRAITEMENT DES INFECTIONS PAR LEISHMANIA

(30) Priorität: 24.10.2003 EP 03090368
(43) Veröffentlichungstag der Anmeldung: 18.01.2006
(73) Patentinhaber: Mologen AG, 14195 Berlin (DE)
(72) Erfinder: WITTIG, Burghardt, 14129 Berlin (DE); FUERTES-LOPEZ, Laura, E-28003 Madrid (ES); TIMON-JIMENEZ, Marcos, E-28200 San Lorenzo de El Escorial (ES)
(74) Vertreter: Boeckh, Tobias
(86) Internationale Anmeldenummer: PCT/DE2004/002383
(87) Internationale Veröffentlichungsnummer: WO 2005/039633

(56) Entgegenhaltungen:
- WO-A-02/098359
- WO-A-03/031469
- LOPEZ-FUERTES L ET AL: "DNA vaccination with linear minimalistic (MIDGE) vectors confers protection against Leishmania major infection in mice" VACCINE, BUTTERWORTH SCIENTIFIC. GUILDFORD, GB, Bd. 21, Nr. 3-4, 13. Dezember 2002 (2002-12-13), Seiten 247-257, XP004394510 ISSN: 0264-410X
- SKEIKY Y A W ET AL: "Protective efficacy of a tandemly linked, multi-subunit recombinant leishmanial vaccine (Leish-111f) formulated in MPL adjuvant" VACCINE, BUTTERWORTH SCIENTIFIC. GUILDFORD, GB, Bd. 20, Nr. 27-28, 10. September 2002 (2002-09-10), Seiten 3292-3303, XP004378524 ISSN: 0264-410X
- MENDEZ S ET AL: "Optimization of DNA vaccination against cutaneous leishmaniasis" VACCINE, BUTTERWORTH SCIENTIFIC. GUILDFORD, GB, Bd. 20, Nr. 31-32, 1. November 2002 (2002-11-01), Seiten 3702-3708, XP004388612 ISSN: 0264-410X
- RAMREZ J R ET AL: "Attenuated Toxoplasma gondii ts-4 mutants engineered to express the Leishmania antigen KMP-11 elicit a specific immune response in BALB/c mice" VACCINE, BUTTERWORTH SCIENTIFIC. GUILDFORD, GB, Bd. 20, Nr. 3-4, 12. November 2001 (2001-11-12), Seiten 455-461, XP004310152 ISSN: 0264-410X
- COTE-SIERRA JAVIER ET AL: "Bacterial lipoprotein-based vaccines induce tumor necrosis factor-dependent type 1 protective immunity against Leishmania major" INFECTION AND IMMUNITY, Bd. 70, Nr. 1, Januar 2002 (2002-01), Seiten 240-248, XP002317814 ISSN: 0019-9567

### SEQUENCE LISTING

<110> Mologen AG
<120> MITTEL ZUR BEHANDLUNG VON INFEKTIONEN MIT LEISHMANIOSE
<130> XI 1827-03
<150> EP 03090368.6
   <151> 2004-10-22
<150> EP 03090368.6
   <151> 2003-10-22
<160> 36
<170> PatentIn version 3.3
<210> 1
   <211> 279
   <212> DNA
   <213> Leishmania infantum
<220>
   <221> misc_feature
   <223> DNA Sequenz des Leishmania infantum kinetoplastid membrane
   protein 11 Gens (kmp-11)
<400> 1
   atggccacca cgtacgagga gttttcggcg aagctggacc gcctggatga ggagttcaac      60
   aggaagatgc aggagcagaa cgccaagttc tttgcggaca agccggatga gtcgacgctg     120
   tcgcccgaga tgaaggagca ctacgagaag ttcgagcgca tgatcaagga acacacagag     180
   aagttcaaca agaagatgca cgagcactcg gagcacttca agcagaagtt cgccgagctg     240
   ctcgagcagc agaaggctgc gcagtaccca tccaagtaa                            279
<210> 2
   <211> 92
   <212> PRT
   <213> Leishmania infantum
<220>
   <221> MISC_FEATURE
   <223> Protein Sequenz des Leishmania infantum kinetoplastid membrane protein 11 (kmp-11)
<400> 2
   Met Ala Thr Thr Tyr Glu Glu Phe Ser Ala Lys Leu Asp Arg Leu Asp
   1               5                   10                  15
   Glu Glu Phe Asn Arg Lys Met Gln Glu Gln Asn Ala Lys Phe Phe Ala
               20                  25                  30
   Asp Lys Pro Asp Glu Ser Thr Leu Ser Pro Glu Met Lys Glu His Tyr
           35                  40                  45
   Glu Lys Phe Glu Arg Met Ile Lys Glu His Thr Glu Lys Phe Asn Lys
       50                  55                  60
   Lys Met His Glu His Ser Glu His Phe Lys Gln Lys Phe Ala Glu Leu
   65                  70                  75                  80
   Leu Glu Gln Gln Lys Ala Ala Gln Tyr Pro Ser Lys
                   85                  90
<210> 3
   <211> 600
   <212> DNA
   <213> Leishmania infantum
<220>
   <221> misc_feature
   <223> DNA Sequenz des Leishmania infantum Antigene thiol-specific antioxidant protein Gens (TSA)
<400> 3
   atgtcctgcg gtaacgccaa gatcaactgt cccgcgccgc ccttcgagga ggtggcgctc     60
   atgcccaacg gcagcttcaa gaagatcagc ctcgccgcct acaagggcaa gtgggtcgtg    120
   ctcttcttct acccgctcga cttcaccttc gtgtgcccga cagagatcat cgcgttctcc    180
   gaaaacgtga gtcgcttcaa cgagctcaac tgcgaggtcc tcgcgtgctc catggacagc    240
   gagtacgcgc acctgcagtg gacgctgcag gaccgcaaga agggcggcct cggcgccatg    300
   gcgattccaa tgctggccga caagaccaag agtatcgctc gtgcctacgg cgtgctggag    360
   gagaaacagg gcgtggccta ccgcggtcta ttcatcatcg accccaatgg catggtgcgc    420
   cagatcaccg tcaacgacat gccggtgggc cgcaacgtgg aggaggttct gcgcctgctg    480
   gaggcttttc agttcgtgga gaagcacggc gaggtgtgcc ccgcgaactg gaagaagggc    540
   gcccccacga tgaagccgga gccgaaggcg tctgtcgaag gatacttcag caagcagtaa    600
<210> 4
   <211> 199
   <212> PRT
   <213> Leishmania infantum
<220>
   <221> MISC_FEATURE
   <223> Protein DNA Sequenz des Leishmania infantum thiol-specific antioxidant Proteins (TSA)
<400> 4
   Met Ser Cys Gly Asn Ala Lys Ile Asn Cys Pro Ala Pro Pro Phe Glu
   1               5                   10                  15
   Glu Val Ala Leu Met Pro Asn Gly Ser Phe Lys Lys Ile Ser Leu Ala
               20                  25                  30
   Ala Tyr Lys Gly Lys Trp Val Val Leu Phe Phe Tyr Pro Leu Asp Phe
           35                  40                  45
   Thr Phe Val Cys Pro Thr Glu Ile Ile Ala Phe Ser Glu Asn Val Ser
       50                  55                  60
   Arg Phe Asn Glu Leu Asn Cys Glu Val Leu Ala Cys Ser Met Asp Ser
   65                  70                  75                  80
   Glu Tyr Ala His Leu Gln Trp Thr Leu Gln Asp Arg Lys Lys Gly Gly
                   85                  90                  95
   Leu Gly Ala Met Ala Ile Pro Met Leu Ala Asp Lys Thr Lys Ser Ile
               100                 105                 110
   Ala Arg Ala Tyr Gly Val Leu Glu Glu Lys Gln Gly Val Ala Tyr Arg
           115                 120                 125
   Gly Leu Phe Ile Ile Asp Pro Asn Gly Met Val Arg Gln Ile Thr Val
       130                 135                 140
   Asn Asp Met Pro Val Gly Arg Asn Val Glu Glu Val Leu Arg Leu Leu
   145                 150                 155                 160
   Glu Ala Phe Gln Phe Val Glu Lys His Gly Glu Val Cys Pro Ala Asn
                   165                 170                 175
   Trp Lys Lys Gly Ala Pro Thr Met Lys Pro Glu Pro Lys Ala Ser Val
               180                 185                 190
   Glu Gly Tyr Phe Ser Lys Gln
           195
<210> 5
   <211> 1800
   <212> DNA
   <213> Artificial
<220>
   <223> DNA Sequenz des codon-optimierten Leishmania infantum Antigen gp63
<400> 5
   atgtctgtgg actcctcctc cacccacaga cacagatctg tggctgccag actggtgaga      60
   ctggctgcag ctggagctgc tgtgattgct gctgtgggca cagctgctgc ctgggcccat     120
   gctggagctg tgcagcacag atgcatccat gatgccatgc aggccagagt gagacagtct     180
   gtggccagac accacacagc cccaggagct gtgtctgctg tgggcctgcc ctatgtgacc 240
   ctggacacag ctgcagctgc agacagaaga ccaggctctg cccccacagt ggtgagagct 300
   gccaactggg gagccctgag gattgctgtg tccacagagg acctgacaga cccagcctac 360
   cactgtgcca gagtgggcca gcacatcaag agaagactgg gaggagtgga catctgcaca 420
   gctgaggaca tcctgacaga tgagaagaga gacatcctgg tgaagcacct gatcccccag 480
   gccctgcagc tgcacacaga gagactgaaa gtgagacagg tgcaggacaa gtggaaggtg 540
   acaggcatgg gagatgatgt gtgctctgac ttcaaggtgc ccccagccca catcacagat 600
   ggcctgtcca acacagactt tgtgatgtat gtgacctctg tgccctctga ggagggagtg 660
   ctggcctggg ccaccatctg ccaggtgttc tctgatggcc acccaaccgt gggagtgatc 720
   aacatcccag ctgccaacat tgcctccaga tatgaccagc tggtgaccag agtggtgacc 780
   catgagatgg cccatgccct gggcttctct gtgggcttct ttgagggagc cagaatcctg 840
   gagtccatct ccaatgtgag acacaaggac tttgatgtgc cagtgatcaa ctcctccact 900
   gctgtggcca aggccagaga gcagtatggc tgtgacaccc tggagtacct ggagattgag 960
   gaccagggag gagctggctc tgctggctcc cacatcaaga tgagaaatgc ccaggatgag 1020
   ctgatggccc cagctgcagc tgcaggctac tactctgccc tgaccacggc catcttccag 1080
   gacctgggct tctaccaggc tgacttctcc aaggctgagg tgatgccctg gggcagaaat 1140
   gctggctgtg ccttcctgtc tgagaagtgc atggagagaa acatcaccga gtggccagcc 1200
   atgttctgca atgagaatga ggtgaccatg agatgcccca cctccagact gtccctgggc 1260
   aagtgtggag tgaccagaca cccagacctg cccccctact ggcagtactt cacagacccc 1320
   tccctggctg gcatctctgc cttcatggac tgctgcccag tggcggagcc ctatggagat 1380
   ggctcctgtg cccagagagc ctctgaggct ggagccccct tcaagggctt caatgtgttc 1440
   tctgatgctg cccgatgcat tgatggagcc ttcagaccca agacctccca tggcatcatc 1500
   aagtcctatg ctggcctgtg tgccaatgtg agatgtgaca cagccaccag aacctactct 1560
   gtgcaggtgc atggaggctc tggctatgcc aactgcaccc caggcctgag agtggagctg 1620
   tccacagtgt cctctgcctt tgaggaggga ggctacatca cctgcccccc ctatgtggag 1680
   gtgtgccagg gcaatgtgca ggctgccaag gatggaggca atgcagctgc tggcagaaga 1740
   ggccccagag ctgctgccac agccctgctg gtggctgccc tgctggctgt ggccctgtag 1800
<210> 6
   <211> 599
   <212> PRT
   <213> Artificial
<220>
   <223> Protein Sequenz des codon-optimierten Leishmania infantum Antigen gp63
<400> 6
   Met Ser Val Asp Ser Ser Ser Thr His Arg His Arg Ser Val Ala Ala
   1 5 10 15
   Arg Leu Val Arg Leu Ala Ala Ala Gly Ala Ala Val Ile Ala Ala Val
   20 25 30
   Gly Thr Ala Ala Ala Trp Ala His Ala Gly Ala Val Gln His Arg Cys
   35 40 45
   Ile His Asp Ala Met Gln Ala Arg Val Arg Gln Ser Val Ala Arg His
   50 55 60
   His Thr Ala Pro Gly Ala Val Ser Ala Val Gly Leu Pro Tyr Val Thr
   65 70 75 80
   Leu Asp Thr Ala Ala Ala Ala Asp Arg Arg Pro Gly Ser Ala Pro Thr
   85 90 95
   Val Val Arg Ala Ala Asn Trp Gly Ala Leu Arg Ile Ala Val Ser Thr
   100 105 110
   Glu Asp Leu Thr Asp Pro Ala Tyr His Cys Ala Arg Val Gly Gln His
   115 120 125
   Ile Lys Arg Arg Leu Gly Gly Val Asp Ile Cys Thr Ala Glu Asp Ile
   130 135 140
   Leu Thr Asp Glu Lys Arg Asp Ile Leu Val Lys His Leu Ile Pro Gln
   145 150 155 160
   Ala Leu Gln Leu His Thr Glu Arg Leu Lys Val Arg Gln Val Gln Asp
   165 170 175
   Lys Trp Lys Val Thr Gly Met Gly Asp Asp Val Cys Ser Asp Phe Lys
   180 185 190
   Val Pro Pro Ala His Ile Thr Asp Gly Leu Ser Asn Thr Asp Phe Val
   195 200 205
   Met Tyr Val Thr Ser Val Pro Ser Glu Glu Gly Val Leu Ala Trp Ala
   210 215 220
   Thr Ile Cys Gln Val Phe Ser Asp Gly His Pro Thr Val Gly Val Ile
   225 230 235 240
   Asn Ile Pro Ala Ala Asn Ile Ala Ser Arg Tyr Asp Gln Leu Val Thr
   245 250 255
   Arg Val Val Thr His Glu Met Ala His Ala Leu Gly Phe Ser Val Gly
   260 265 270
   Phe Phe Glu Gly Ala Arg Ile Leu Glu Ser Ile Ser Asn Val Arg His
   275 280 285
   Lys Asp Phe Asp Val Pro Val Ile Asn Ser Ser Thr Ala Val Ala Lys
   290 295 300
   Ala Arg Glu Gln Tyr Gly Cys Asp Thr Leu Glu Tyr Leu Glu Ile Glu
   305 310 315 320
   Asp Gln Gly Gly Ala Gly Ser Ala Gly Ser His Ile Lys Met Arg Asn
   325 330 335
   Ala Gln Asp Glu Leu Met Ala Pro Ala Ala Ala Ala Gly Tyr Tyr Ser
   340 345 350
   Ala Leu Thr Thr Ala Ile Phe Gln Asp Leu Gly Phe Tyr Gln Ala Asp
   355 360 365
   Phe Ser Lys Ala Glu Val Met Pro Trp Gly Arg Asn Ala Gly Cys Ala
   370 375 380
   Phe Leu Ser Glu Lys Cys Met Glu Arg Asn Ile Thr Glu Trp Pro Ala
   385 390 395 400
   Met Phe Cys Asn Glu Asn Glu Val Thr Met Arg Cys Pro Thr Ser Arg
   405 410 415
   Leu Ser Leu Gly Lys Cys Gly Val Thr Arg His Pro Asp Leu Pro Pro
   420 425 430
   Tyr Trp Gln Tyr Phe Thr Asp Pro Ser Leu Ala Gly Ile Ser Ala Phe
   435 440 445
   Met Asp Cys Cys Pro Val Ala Glu Pro Tyr Gly Asp Gly Ser Cys Ala
   450 455 460
   Gln Arg Ala Ser Glu Ala Gly Ala Pro Phe Lys Gly Phe Asn Val Phe
   465 470 475 480
   Ser Asp Ala Ala Arg Cys Ile Asp Gly Ala Phe Arg Pro Lys Thr Ser
   485 490 495
   His Gly Ile Ile Lys Ser Tyr Ala Gly Leu Cys Ala Asn Val Arg Cys
   500 505 510
   Asp Thr Ala Thr Arg Thr Tyr Ser Val Gln Val His Gly Gly Ser Gly
   515 520 525
   Tyr Ala Asn Cys Thr Pro Gly Leu Arg Val Glu Leu Ser Thr Val Ser
   530 535 540
   Ser Ala Phe Glu Glu Gly Gly Tyr Ile Thr Cys Pro Pro Tyr Val Glu
   545 550 555 560
   Val Cys Gln Gly Asn Val Gln Ala Ala Lys Asp Gly Gly Asn Ala Ala
   565 570 575
   Ala Gly Arg Arg Gly Pro Arg Ala Ala Ala Thr Ala Leu Leu Val Ala
   580 585 590
   Ala Leu Leu Ala Val Ala Leu
   595
<210> 7
   <211> 939
   <212> DNA
   <213> Leishmania infantum
<220>
   <221> misc_feature
   <223> DNA Sequenz Leishmania Antigen p36 (LACK)
<400> 7
   atgaactacg agggtcacct gaagggccac cgcggatggg tcacctccct ggcctgcccg 60
   cagcaggcgg ggtcgtacat caaggtggtg tcgacgtcgc gcgatggcac ggccatctcg 120
   tggaaagcca accccgaccg ccacagcgtg gacagcgact acggtctgcc gagccaccgc 180
   ctcgagggcc acaccggctt cgtgtcgtgt gtgtcgctgg cccacgccac cgactacgcg 240
   ctgaccgcgt cctgggaccg ctccatccgc atgtgggacc tgcgcaatgg ccagtgccag 300
   cgcaagttcc tgaagcacac caaggacgtg ctcgccgtcg ccttctcgcc ggacgaccgc 360
   ctgatcgtgt ccgcgggccg cgacaacgtg atccgcgtgt ggaacgtggc gggcgagtgc 420
   atgcacgagt tcctgcgcga cggccacgag gactgggtga gcagcatctg tttctcgccg 480
   tcgctggagc atccgatcgt ggtgtccggc agctgggaca acaccatcaa ggtatggaac 540
   gtgaacgggg gcaagtgtga gcgcacgctc aagggccaca gcaactacgt gtccacggtg 600
   acggtgtcgc cagacgggtc gctgtgcgcg tccggcggca aggacggcgc ggcgctgctg 660
   tgggacctga gcaccggcga gcagctgttc aagatcaacg tggagtcgcc catcaaccag 720
   atcgccttct cgcccaaccg cttctggatg tgcgtcgcga cggagaggtc cctgtccgtg 780
   tacgacctgg agagcaaggc tgtgattgcg gagctgacgc cggacggcgc gaagccgtcc 840
   gagtgcatct ccattgcctg gtccgccgac ggcaacactc tgtactccgg tcacaaggac 900
   aacctgatcc gcgtgtggtc catctccgac gccgagtaa 939
<210> 8
   <211> 312
   <212> PRT
   <213> Leishmania infantum
<220>
   <221> MISC_FEATURE
   <223> Protein Sequenz Leishmania Antigen p36 (LACK)
<400> 8
   Met Asn Tyr Glu Gly His Leu Lys Gly His Arg Gly Trp Val Thr Ser
   1 5 10 15
   Leu Ala Cys Pro Gln Gln Ala Gly Ser Tyr Ile Lys Val Val Ser Thr
   20 25 30
   Ser Arg Asp Gly Thr Ala Ile Ser Trp Lys Ala Asn Pro Asp Arg His
   35 40 45
   Ser Val Asp Ser Asp Tyr Gly Leu Pro Ser His Arg Leu Glu Gly His
   50 55 60
   Thr Gly Phe Val Ser Cys Val Ser Leu Ala His Ala Thr Asp Tyr Ala
   65 70 75 80
   Leu Thr Ala Ser Trp Asp Arg Ser Ile Arg Met Trp Asp Leu Arg Asn
   85 90 95
   Gly Gln Cys Gln Arg Lys Phe Leu Lys His Thr Lys Asp Val Leu Ala
   100 105 110
   Val Ala Phe Ser Pro Asp Asp Arg Leu Ile Val Ser Ala Gly Arg Asp
   115 120 125
   Asn Val Ile Arg Val Trp Asn Val Ala Gly Glu Cys Met His Glu Phe
   130 135 140
   Leu Arg Asp Gly His Glu Asp Trp Val Ser Ser Ile Cys Phe Ser Pro
   145 150 155 160
   Ser Leu Glu His Pro Ile Val Val Ser Gly Ser Trp Asp Asn Thr Ile
   165 170 175
   Lys Val Trp Asn Val Asn Gly Gly Lys Cys Glu Arg Thr Leu Lys Gly
   180 185 190
   His Ser Asn Tyr Val Ser Thr Val Thr Val Ser Pro Asp Gly Ser Leu
   195 200 205
   Cys Ala Ser Gly Gly Lys Asp Gly Ala Ala Leu Leu Trp Asp Leu Ser
   210 215 220
   Thr Gly Glu Gln Leu Phe Lys Ile Asn Val Glu Ser Pro Ile Asn Gln
   225 230 235 240
   Ile Ala Phe Ser Pro Asn Arg Phe Trp Met Cys Val Ala Thr Glu Arg
   245 250 255
   Ser Leu Ser Val Tyr Asp Leu Glu Ser Lys Ala Val Ile Ala Glu Leu
   260 265 270
   Thr Pro Asp Gly Ala Lys Pro Ser Glu Cys Ile Ser Ile Ala Trp Ser
   275 280 285
   Ala Asp Gly Asn Thr Leu Tyr Ser Gly His Lys Asp Asn Leu Ile Arg
   290 295 300
   Val Trp Ser Ile Ser Asp Ala Glu
   305 310
<210> 9
   <211> 7
   <212> PRT
   <213> Simian virus 40
<220>
   <221> MISC_FEATURE
   <223> Kernlokalisationssequenz
<400> 9
   Pro Lys Lys Lys Arg Lys Val
   1 5
<210> 10
   <211> 11
   <212> PRT
   <213> Human immunodeficiency virus type 1
<220>
   <221> MISC_FEATURE
   <223> Langes T-Peptidfragment des HIV-1 Genprodukts TAT
<400> 10
   Tyr Gly Arg Lys Lys Arg Arg Gln Arg Arg Arg
   1 5 10
<210> 11
   <211> 33
   <212> DNA
   <213> Artificial
<220>
   <223> 1. PCR-Primer links; synthetisches Oligo
<400> 11
   ttatatggta ccatgaacat acgagggtca cct 33
<210> 12
   <211> 42
   <212> DNA
   <213> Artificial
<220>
   <223> 1. PCR-Primer rechts; synthetisches Oligo
<400> 12
   ttatatgagc tcagaagaca cggacaggga cctcttccgt cg 42
<210> 13
   <211> 33
   <212> DNA
   <213> Artificial
<220>
   <223> 2. PCR-Primer links; synthetisches Oligo
<400> 13
   ttatatggta ccatgaacat acgagggtca cct 33
<210> 14
   <211> 33
   <212> DNA
   <213> Artificial
<220>
   <223> 2.PCR-Primer rechts; synthetisches Oligo
<400> 14
   ttatatgagc tcttactcgg ccgtcggaga tgg 33
<210> 15
   <211> 30
   <212> DNA
   <213> Artificial
<220>
   <223> Kmp-11 PCR-Primer links; synthetisches Oligo
<400> 15
   attataggta ccatggccac cacgtacgag 30
<210> 16
   <211> 32
   <212> DNA
   <213> Artificial
<220>
   <223> Kmp-11 PCR-Primer rechts; synthetisches Oligo
<400> 16
   ttaattctcg agttacttgg atgggtactg cg 32
<210> 17
   <211> 36
   <212> DNA
   <213> Artificial
<220>
   <223> TSA PCR-Primer links; synthetisches Oligo
<400> 17
   aattatggta ccatgtcctg cggtaacgcc aagatc 36
<210> 18
   <211> 39
   <212> DNA
   <213> Artificial
<220>
   <223> TSA PCR-Primer rechts; synthetisches Oligo
<400> 18
   aatatactcg agttactgct tgctgaagta tccttcgac 39
<210> 19
   <211> 22
   <212> DNA
   <213> Artificial
<220>
   <223> TSA linker Mutationsprimer; synthetisches Oligo
<400> 19
   taccgcggtc tcttcatcat cg 22
<210> 20
   <211> 32
   <212> DNA
   <213> Artificial
<220>
   <223> TSA rechter Mutationsprimer; synthetisches Oligo
<400> 20
   attgggggtc tcgatgaata gaccgcggta gg 32
<210> 21
   <211> 25
   <212> DNA
   <213> Artificial
<220>
   <223> gp63 Fragment 1 linker Primer; synthetisches Oligo
<400> 21
   attattggta ccatgtctgt ggact 25
<210> 22
   <211> 31
   <212> DNA
   <213> Artificial
<220>
   <223> gp63 Fragment 1 rechter Primer; synthetisches Oligo
<400> 22
   ttatatggtc tctctcaggg ctccccagtt g 31
<210> 23
   <211> 36
   <212> DNA
   <213> Artificial
<220>
   <223> gp63 Fragment 2 linker Primer; synthetisches Oligo
<400> 23
   attataggtc tcctgagaat tgctgtgtcc acagag 36
<210> 24
   <211> 34
   <212> DNA
   <213> Artificial
<220>
   <223> gp63 Fragment 2 rechter Primer; synthetisches Oligo
<400> 24
   ttatatggtc tcacagaggc cacatacatc acaa 34
<210> 25
   <211> 43
   <212> DNA
   <213> Artificial
<220>
   <223> gp63 Fragment 3 linker Primer; synthetisches Oligo
<400> 25
   tattatggtc tcctctgtgc cctctgagga gggagtgctg gcc 43
<210> 26
   <211> 34
   <212> DNA
   <213> Artificial
<220>
   <223> gp63 Fragment 3 rechter Primer; synthetisches Oligo
<400> 26
   aattatggtc tcctcaatct ccaggtactc cagg 34
<210> 27
   <211> 31
   <212> DNA
   <213> Artificial
<220>
   <223> gp63 Fragment 4 linker Primer; synthetisches Oligo
<400> 27
   attataggtc tcattgagga ccagggagga g 31
<210> 28
   <211> 34
   <212> DNA
   <213> Artificial
<220>
   <223> gp63 Fragment 4 rechter Primer; synthetisches Oligo
<400> 28
   taatatggtc tcgtgtctgg tcactccaca cttg 34
<210> 29
   <211> 31
   <212> DNA
   <213> Artificial
<220>
   <223> gp63 Fragment 5 linker Primer; synthetisches Oligo
<400> 29
   attataggtc tcagacaccc agacctgccc c 31
<210> 30
   <211> 32
   <212> DNA
   <213> Artificial
<220>
   <223> gp63 Fragment 5 rechter Primer; synthetisches Oligo
<400> 30
   ttatatggtc tcggggtgca gttggcatag cc 32
<210> 31
   <211> 32
   <212> DNA
   <213> Artificial
<220>
   <223> gp63 Fragement 6 linker Primer; synthetisches Oligo
<400> 31
   atatatggtc tccaccccag gcctgagagt gg 32
<210> 32
   <211> 34
   <212> DNA
   <213> Artificial
<220>
   <223> gp63 Fragment 6 rechter Primer; synthetisches Oligo
<400> 32
   taatatgagc tcctacaggg ccacagccag cagg 34
<210> 33
   <211> 25
   <212> DNA
   <213> Artificial
<220>
   <223> gp63 Gesamtsequenz linker Primer; synthetisches Oligo
<400> 33
   attattggta ccatgtctgt ggact 25
<210> 34
   <211> 34
   <212> DNA
   <213> Artificial
<220>
   <223> gp63 Gesamtsequenz rechter Primer; synthetisches Oligo
<400> 34
   taatatgagc tcctacaggg ccacagccag cagg 34
<210> 35
   <211> 20
   <212> DNA
   <213> Artificial
<220>
   <223> ODN1; synthetisches Oligo
<220>
   <221> misc_feature
   <222> (12)..(12)
   <223> T = chemisch modifiziertes Thymin
<400> 35
   gggagtccag ttttctggac 20
<210> 36
   <211> 20
   <212> DNA
   <213> Artificial
<220>
   <223> ODN 2; synthetisches Oligo
<400> 36
   aggggtccag ttttctggac 20

## Beschreibung

Die Erfindung betrifft die Verwendung einer Kombination von DNA-Expressionskonstrukten zur Herstellung eines Arzneimittels zur Immunisierung gegen Leishmaniose-Infektionen, sowie eine entsprechende Vakzine. Die DNA-Expressionskonstrukte selbst sind ebenfalls Gegenstand der Erfindung.

Leishmanien sind trypanosomatide Flagellaten aus der Ordnung Kinetoplastida, die durch weibliche blutsaugende Sandmücken der Gattung Phlebotomus und Lutzomyia auf verschiedene Säugetierspezies und auf den Menschen übertragen werden. Leishmaniosen sind Erkrankungen mit verschiedenen klinischen Krankheitsbildern, die ein bedeutendes Gesundheitsproblem darstellen. Die WHO schätzt, dass weltweit ca. 12 Millionen Menschen von der Krankheit betroffen sind. Ca. 2 bis 9% aller HIV-Patienten erkranken an viceraler Leishmaniose, damit ist dies die dritt häufigste parasitäre Krankheit, an der HIV-positive Personen erkranken. Während bei den mukokutanen und kutanen Leishmaniosen erhebliche Gewebedestruktionen auftreten, endet eine unbehandelte viszerale Leishmaniose (Kala-Azar) meist tödlich.

Für die Behandlung der Erkrankung stehen nur wenige klinisch erprobte Medikamente zur Verfügung. So werden seit etwa 60 Jahren zur Therapie der viszeralen Leishmaniose Chemotherapeutika - meist Verbindungen des Schwermetalls Antimon - eingesetzt. Die zum Teil massive Toxizität der meisten dieser Präparate begrenzt jedoch deren Einsatz. Zudem haben die Leishmanien in vielen Gebieten Resistenzen gegen Antimonpräparate entwickelt (J Postgrad Med. 2003 Jan-Mar; 49(1):61-8).

Eine verträgliche und protektive Routineimpfung gibt es bislang nicht.

Da Personen, die die Infektion überstanden haben, eine starke Immunität gegen eine spätere Infektion entwickeln, sollte die Entwicklung eines wirksamen Impfschutzes möglich sein.

Die Impfung bzw. Immuntherapie von Leishmaniose, deren Ursache intrazelluläre Parasiten sind, sollte durch die Erzeugung einer Th1-typischen Immunreaktion möglich sein. Im Stand der Technik wird vielfach auf die Bedeutung der Erzeugung einer Th1-Antwort in der Therapie oder Prävention von Leishmaniose hingewiesen. (Handman et al., J Immunol 160: 3949-57, Gurunathan et al., Nature Med: 4(12): 1409-15). Zur Förderung der Auslösung einer Th1-typischen Immunantwort wird auf das kostimulatorische Zytokin IL-12 als unerlässliches Adjuvanz verwiesen (Parker et al., J. Immunol. 140: 896-902).

Zusätzlich können immunstimulatorische Nukleinsäuresequenzen (ISS) als Adjuvanz eingesetzt werden. Die CpG-Motive der ISS bewirken eine Erhöhung der Aktivität von NK-Zellen und Makrophagen sowie eine starke Stimulation der zellulären TH1-Immunantwort. Bevorzugt können kovalent geschlossene ISS mit einer Länge von 30 bp eingesetzt werden, wie sie beispielsweise in der EP 1 196 178 A1 beschrieben sind.

Verschiedene Antigene wurden in experimentellen Impfprotokollen in Mäusen getestet. Die immunologische Reaktion in Mäusen auf diese Infektion, scheint der im Menschen und wahrscheinlich auch der in Hunden zu gleichen (Cox, Int. J. Parasitol. 263: 1147-1157). Verwendete Antigene waren das gp 63 (Scott et al., J. Exp Med.168: 1675-1684), gp 46 (McMahon-Pratt et al., Infection and Immunity 61: 3351-3359), p-4 und p-8 (Scott et al., Immunology 99: 615-624) sowie das p36 oder auch als LACK bezeichnete Antigen (Gonzales-Aseguinolaza et al., Eur. J. Biochem. 259: 909-916). Das erfolgreichste Impfregime, Erstimmunisation mit p36 Protein, Zweitimmunisation mit Vaccinia Virus, kodierend für p36 und IL-12, führte zu einer durchschnittlichen Verkleinerung der Läsionen um 52% im Vergleich zu ungeimpften Mäusen (Gonzalo et al., Microbes and Infection: 3 (9): 701-711).

Neben einem nur 52%igem Schutz wurden in dem zitierten Versuch Vaccinia-Viren als Genfähren eingesetzt. Dabei handelt es sich um virale Vektoren, die auf Grund ihrer hohen Transfektionseffizienz immer noch die am häufigsten eingesetzten Genfähren darstellen. Es ist jedoch bekannt, dass ein hohes Risiko einer zytotoxischen Reaktion des Wirtsorganismus auf die transfizierten Zellen besteht. So führte die Anwendung einer hohen Dosis eines Adenovirus bei einem klinischen Versuch zum Tod des Patienten; offensichtlich war die Ursache dafür eine starke Überreaktion des Immunsystems (Lehrman, 1999, Nature 401: 517-518). Weiterhin ist durch die Instabilität des attenuierten Impfstammes die Rückverwandlung in einen virulenten Stamm nicht auszuschließen. Außerdem können die viralen Bestandteile selbst immunogen wirken, was zu einer Herabsetzung ihrer Wirksamkeit durch das Immunsystems des Patienten führt.

In eigenen Arbeiten der Anmelderin wurden BALB/c Mäuse mit minimalistischen Expressionsvektoren kodierend für das p36 LACK-Antigen immunisiert. Dabei wurden verschiedene Impfschemata angewendet. Im Rahmen dieser Studie konnte in einer Gruppe ein 57%iger Schutz vor einer Infektion mit Leishmania major erreicht werden (L. Lopez-Fuertes et al., 2002, Vaccine 21: 247-257).

Gurunathan et al. setzten das p36 LACK-Antigen, durch eukaryotische Expressionsvektoren kodiert, in Impfversuchen in Mäusen ein (J. Exp. Med., Vol 186, No. 7, (1997): 1137-1147).

In anderen Ansätzen wurden verschiedene Antigenkombinationen eingesetzt. Mit einem Gemisch an Plasmid-DNA, kodierend für TSA und LmST11, konnte so die Größe der Läsionen über einen bestimmten Zeitraum nach der Infektion kleingehalten werden (A. Campos-Neto et al., 2002, Infection and Immunity: 2828-2836).

Die Dreierkombination der Antigene LACK, LmST11 und TSA konnte das Auftreten von dermalen Läsionen nach der Infektion zum großen Teil verhindern und führte zu einem über mehrere Wochen anhaltenden Schutz (S. Mendez et al., 2001, J. of Immunology 166: 5122-5128).

Allen zitierten Versuchen ist gemeinsam, dass durch sie nur ein teilweiser und damit unzureichender Schutz vor einer Infektion mit Leishmaniose erreichbar war. Ebenso wurden die Impfstoffkombinationen nachteilhafterweise nicht in Hunden, dem Hauptüberträger, sondern in Mäusen erprobt. Ein weiterer Nachteil ist, dass die eingesetzten Genfähren Plasmide sind. Plasmide werden durch bakterielle Fermentation gewonnen. Dadurch enthalten sie neben dem gewünschten Gen auch die für ihre Vervielfältigung und Selektion notwendige DNA, üblicherweise Resistenzgene gegen bei der Fermentation verwendete Antibiotika. Bei der Verwendung von Genexpressionskonstrukten auf Basis von Plasmid-DNA besteht dadurch das inhärente Risiko der Verbreitung von Antibiotikaresistenzgenen, welches insbesondere bei Schutzimpfungskampagnen nicht vertretbar ist. In der medizinischen Praxis stehen die beschriebenen Nachteile plasmidbasierter Expressionsvektoren ihrer breiten Anwendung massiv entgegen.

Ausgehend von diesem Stand der Technik ist es daher Aufgabe der vorliegenden Erfindung, ein DNA-Expressionskonstrukt bzw. auch mehrere DNA-Expressionskonstrukte zur Verfügung zu stellen, welche zur Herstellung eines Arzneimittels zur effizienten Immunisierung gegen Leishmaniose verwendet werden können.

Die Aufgabe wird durch die Merkmale der unabhängigen Ansprüche gelöst.

Als DNA-Expressionskonstrukte werden bevorzugt werden erfindungsgemäß minimalistische, immunologisch definierte Genexpressionskonstrukte verwendet, die im Folgenden als MIDGE-Vektoren bezeichnet werden (MIDGE^{®}: MINIMALISTIC IMMUNOLOGICALLY DEFINED GENE EXPRESSION VECTORS, vgl. EP 0 941 318 B1, US 6,451,593 B1). Die MIDGE-Vektoren haben den Vorteil, dass durch sie auf Strukturen verzichtet werden kann, die für die therapeutische Wirkung nicht essentiell sind.

Erfindungsgemäß ist insbesondere vorgesehen, dass das immunogene Antigen p36 *LACK* in Kombination mit dem Leishmania infantum *thiol-specific antioxidant protein* Antigen (TSA), dem Leishmania infantum *kinetoplastid membrane protein 11* Antigen (Kmp-11) und dem Leishmania infantum *Glykoprotein 63* (gp63) Antigen zur Erzeugung einer Immunantwort verwendet wird.

Infolgedessen ist die Verwendung eines DNA-Expressionskonstruktes zur Herstellung eines Arzneimittels zur Immunisierung gegen Leishmaniose-Infektionen vorgesehen, wobei besagtes DNA-Expressionskonstrukt ein oder mehrere kodierende Nukleinsäuresequenzen enthält, welche zur Expression der *Leishmania infantum* Antigene *thiol-specific antioxidant protein Gen* (TSA), Glykoprotein gp63 und *kinetoplastid membrane protein 11* (Kmp-11) führen.

Bevorzugt ist aber auch die Verwendung von zwei oder drei DNA-Expressionskonstrukten zur Herstellung eines Arzneimittels zur Immunisierung gegen Leishmaniose-Infektionen, wobei besagte DNA-Expressionskonstrukte jeweils ein oder mehrere kodierende Nukleinsäuresequenzen enthalten, die gemeinsam zur Expression der *Leishmania infantum* Antigene *thiol-specific antioxidant protein Gen* (TSA), Glykoprotein gp63 und *kinetoplastid membrane protein* 11 (Kmp-11) führen.

Erfindungsgemäß ist vorgesehen, dass wenigstens zwei der Leishmania infantum Antigene als Fusionsprotein exprimiert werden. Dabei kann es sich einerseits um das Fusionsprotein - als Expressionsprodukt - aus *thiol-specific antioxidant protein Gen* (TSA) und *kinetoplastid membrane protein 11* Gen (Kmp-11) handeln. Dementsprechend ist auch im Hinblick auf die drei unterschiedlichen *Leishmania infantum* Antigene ein Fusionsprotein - als Expressionsprodukt - aus *thiol-specific antioxidant protein Gen* (TSA), Glykoprotein gp63 Gen und *kinetoplastid membrane protein 11* Gen (Kmp-11), bevorzugt.

Hierbei ist jedwede Reihenfolge/Ausrichtung der Gene bzw. der Leserichtung möglich, also z.B. [TSA-KMp-11], aber auch [Kmp-11 - TSA], bzw. [TSA - gp63 - Kmp-11] oder (TSA - Kmp-11 - gp63] oder [Kmp-11 - TSA - gp63] usw. usw..

Es ist demnach also ebenfalls ein Genexpressionskonstrukt kodierend für gegebenenfalls unterschiedliche Fusionsproteine vorgesehen. Das Fusionsprotein besteht wie beschrieben aus einer Kombination von mindestens zwei der benannten Antigene (TSA, Kmp-11). Das Bifusionsprotein wird in Kombination mit dem Antigen gp 63 eingesetzt.

Das Fusionsprotein kann jedoch auch wie beschrieben aus drei Antigenen bestehen. Es handelt sich dann um eine Kombination der Antigene TSA, Kmp-11 und gp63.

Ferner ist eine Verwendung bevorzugt, wobei zusätzlich zu den beschriebenen DNA-Expressionsprodukten - gleichgültig ob dabei Fusionsproteine exprimiert werden oder nicht - ein DNA-Expressionskonstrukt zur Expression des Leishmania Antigens p36 LACK enthalten ist. Das p36 LACK Antigen kann demnach diesem Cocktail optional zugesetzt werden.

Als Genfähre wird eine linear-doppelsträngige kovalent geschlossene MIDGE-Expressionskassette verwendet. Die immunisierenden Polynukleotidsequenzen liegen als Expressionskonstrukte vor, die aus kovalent geschlossenen linearen Desoxyribonukleinsäuremolekülen bestehen, welche einen linearen Doppelstrangbereich aufweisen, wobei die doppelstrangbildenden Einzelstränge durch kurze einzelsträngige Schlaufen aus Desoxyribonukleinsäurenukleotiden verknüpft sind, wobei die doppelstrangbildenden Einzelstränge nur aus der kodierenden Sequenz unter Kontrolle eines im zu impfenden Tier operablen Promotors und einer Terminatorsequenz bestehen. Die Expressionskassette besteht also aus der kodierenden Sequenz, dem Promotor und gegebenenfalls einer Terminationssequenz, so dass das Konstrukt nur die für die Expression des gewünschten Gens notwendige Information enthält, was letztlich die Nachteile der Genfähren viralen Ursprungs vermeidet. Ferner ist erfindungsgemäß vorgesehen, dass das (oder die) DNA-Expressionskonstrukt(e) zur Steigerung der Transfektionseffizienz jeweils mit einem Oligopeptid aus 3 bis 30 Aminosäuren konjugiert ist, wobei das Oligopeptid zur Hälfte aus basischen Aminosäuren aus der Gruppe Arginin und Lysin besteht. Besonders bevorzugt ist.
- die ein Kern-Lokalisierungssignal (Nuclear Localization Signal = NLS) darstellende Peptidsequenz PKKKRKV (Prolin-Lysin-Lysin-Lysin-Arginin-Lysin-Valin) aus dem Simian Virus SV-40. Speziell für das SV-40-NLS wurde gezeigt, dass Proteine bis zu 465 kDa zum Zellkern gesteuert werden (Lanford et al. 1986, Cell 15; 46 (4): 575-82). Diese Fähigkeit des Peptids wurde hier durch Kopplung an DNA für die Verbesserung des Gentransfers genutzt.
- oder das elf Aminosäuren lange T-Peptidfragment YGRKKRRQRRR des HIV-1 Genprodukts TAT.

Es sind demnach Oligopeptide, die die Transfektionseffizienz von DNA Impfstoffen verstärken, beispielsweise kationische Peptide und Proteine, als Bestandteil der Expressionskonstrukte bevorzugt.

Die kodierenden Polynukleotidsequenzen können auch in einem zirkulär doppelsträngigen Expressionsvektor vorliegen.

Es ist bekannt, daß durch Optimierung der Kodonbenutzung (Codon Usage Optimization) innerhalb des Expressionskonstruktes an die präferentiell in Säugern benutzten Kodons die Expression von Proteinen erheblich gesteigert werden kann (Grantham et al., Nucleic Acids Res 1980, 9:1893-912). Um mehr Antigen in-vivo zu exprimieren und damit eine stärkere Immunantwort auszulösen, die sich in einem wirksamen und dauerhaften Schutz vor der Infektion mit Leishmaniose zeigt, wurde die Wildtyp Sequenz von gp63 optimiert. Unter Optimierung soll die Kodon-Anpassung, auch als "Codon-Usage-Optimization" bezeichnet, verstanden werden. Dazu wurde eine Klonierungsstrategie entwickelt, die die Synthese dieser optimierten DNA-Sequenz des gp63 aus Oligonukleotiden ermöglichte.

Erfindungsgemäß wurde die Sequenz des Leishmania infantum gp63 Antigens daher kodonoptimiert (Seq.ID 5). Ein DNA-Expressionskonstrukt, welches diese Sequenz aufweist, ist mithin ebenfalls bevorzugt.

Die erfindungsgemäßen DNA-Expressionskonstrukte dienen zur Herstellung eines Impfstoffes zur Behandlung von Leishmaniose-Infektionskrankheiten und sind Bestandteil einer entsprechenden Vakzine.

Die Vakzine kann immunstimulatorische Nukleinsäuresequenzen (ISS) umfassen. Die immunstimulatorischen Nukleinsäuresequenzen, CpG Motive enthaltend, umfassen einen zirkulären Strang Desoxyribonukleinsäure mit einer teilweise zueinander komplementären, antiparallelen Basensequenz. Die immunstimulatorischen Nukleinsäuresequenzen können hantelförmigsein.

Ebenso ist die Kombination des erfindungsgemäßen Impfstoffes mit transfektionsverbessernden Verbindungen, wie beispielsweise PEI (Polyethylenimin), denkbar.

Zur Transfektion können biologische, chemische und/oder physikalische Methoden eingesetzt werden, die zum Stand der Technik gehören, beispielsweise Transfektion mittels ballistischem Transfer oder Elektroporation. Die Transfektion kann mittels intradermaler Injektion durch Spritzen oder nadelfreier Injektionsgeräte erfolgen.

Das beigefügte Sequenzprotokoll, welches Bestandteil der Anmeldung und vorliegender Beschreibung ist, gibt folgende Sequenzen wieder.

| Seq.ID | Sequenzname/-beschreibung |
|---|---|
| Seq.ID 1 | DNA-Sequenz des Leishmania infantum *kinetoplastid membrane protein 11* Ggens (Kmp-11) |
| Seq.ID 2 | Protein-Sequenz des Leishmania infantum *kinetoplastid membrane protein 11* Antigens (Kmp-11) |
| Seq.ID 3 | DNA-Sequenz des Leishmania infantum *thiol-specikc antioxidant protein* Gens (TSA) |
| Seq.ID 4 | Protein-Sequenz des Leishmania infantum *thiol-specific antioxidant protein* Antigens (TSA) |
| Seq. ID 5 | DNA-Sequenz des kodon-optimierten Leishmania infantum Antigens *Glykoprotein* 63 (gp63) |
| Seq.ID 6 | Protein-Sequenz des kodon-optimierten Leishmania infantum Antigens *Glykoprotein* 63 (gp63) |
| Seq.ID 7 | DNA-Sequenz Leishmania infantum Antigen p36 (LACK) |
| Seq.ID 8 | Protein-Sequenz Leishmania infantum Antigen p36 (LACK) |
| Seq.ID 9 | Kernlokalisationssequenz (Peptidsequenz) des SV40 |
| Seq.ID 10 | Langes T-Peptidfragment des HIV-1 Genprodukts TAT (TAT-Peptid) |
| Seql.D 11 bis Seq.ID 36 | synthetische DNA-Oligomere wie unten beschrieben. |

Weitere vorteilhafte Maßnahmen sind in den übrigen Unteransprüchen enthalten. Die Erfindung wird nachfolgend anhand von Figuren und Ausführungsbeispielen näher beschrieben und diskutiert.

Die überraschende Wirkung der erfindungsgemäßen Kombination von DNA-Expressionskonstrukten und eines Arzneimittels, welches eine solche Kombination enthält, wird anhand der Darstellungen gem. der Figuren deutlich. Dabei bedeuten:
- MIDGE-NLS p36: mit NLS gekoppelter MIDGE kodierend für das p36 LACK Antigen
- MIDGE-NLS TSA: mit NLS gekoppelter MIDGE kodierend für das TSA Antigen
- MIDGE-NLS gp63: mit NLS gekoppelter MIDGE kodierend für das gp63 Antigen
- MIDGE-NLS Kmp-11: mit NLS gekoppelter MIDGE kodierend für das Kmp-11 Antigen
- pMCVp36: Plasmid kodierend für p36 LACK Antigen
- pMCV TSA: Plasmid kodierend für TSA Antigen
- pMCV gp63: Plasmid kodierend für gp63 Antigen
- pMCV Kmp-11: Plasmid kodierend für Kmp-11 Antigen

Es zeigt:

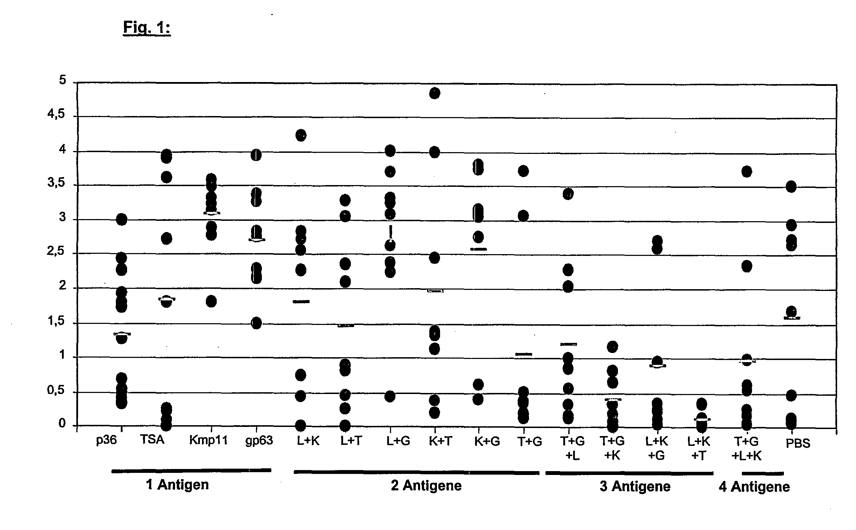

- **Fig. 1:**: Die Entwicklung der Läsionen in Woche 8 nach der Belastungsinfektion in Mäusen. Hierin bedeuten:
L+K: LACK in Kombination mit Kmp-11
L+T: LACK in Kombination mit TSA
L+G: LACK in Kombination mit gp 63
K+T: Kmp-11 in Kombination mit TSA
K+G: Kmp-11 in Kombination mit gp 63
T+G: TSA in Kombination mit gp 63

T+G+L: TSA in Kombination mit gp 63 und LACK
T+G+K: TSA in Kombination mit gp 63 und Kmp-11
L+K+G: LACK in Kombination mit Kmp-11 und gp 63
L+K+T: LACK in Kombination mit Kmp-11 und TSA

T+G+L+K: TSA in Kombination mit gp 63, LACK und Kmp-11

PBS: Kontroll Gruppe: PBS

Die Fig. 1 zeigt die verschiedenen Kombinationen der eingesetzten Antigene in Mäusen. Die dortigen Punkte repräsentieren die Daten der einzelnen Tiere, während die waagerechten Balken den Mittelwert der entsprechenden Gruppe kennzeichnen. Die betreffenden Antigene wurden jeweils einzeln, in sechs verschiedenen Zweierkombinationen, in vier verschiedenen Dreierkombinationen und in einer Viererkombination eingesetzt. As relevanter Parameter für den klinischen Erfolg der Impfung wurde das Wachstum der infektionsbedingten Läsionen in der infizierten Hinterpfote der Tiere gemessen. Die Größenzunahme der Läsionen ist auf der Ordinate in Millimeter aufgetragen. Auf der Abszisse sind die verschiedenen Antigenkombinationen dargestellt. Dabei zeigt sich, das in der Gruppe der Zweierkombinationen die Kombination von TSA mit gp 63 den besten Impfschutz bietet. Den nächstbesten Schutz bietet die Kombination LACK mit TSA. In der Gruppe der Dreierkombination bietet die Gruppe LACK, Kmp-11, TSA den besten Schutz. Die erreichte Impfwirkung zeigt sich in der deutlich verringerten Größe der gebildeten Läsionen. Den zweitbesten Impfschutz bietet die Kombination der Antigene TSA, gp 63 und Kmp-11. In dieser Gruppe wird im Mittel eine Läsionsgröße von 0,4 Millimeter erreicht. Der Wert von 0,5. Millimeter stellt jedoch den Schwellenwert dar, ab dem Läsionen für das menschliche Auge deutlich wahrnehmbar und auswertbar sind. Das bedeutet, dass die Tiere, die mit der Kombination TSA, gp 63 und Kmp-11 wurden, per Definition keine Läsionen zeigten bzw. die festgestellten unter der Nachweisgrenze lagen. Im Vergleich dazu weisen die unbehandelten Mäuse der Kontrollgruppe eine Läsionsgröße von 1,7 Millimetern im Durchschnitt auf. Ebenso trat bei diesen Tieren eine starke Schwellung und Rötung der Pfoten auf, Symptome, die ebenfalls nicht in den geschützten Gruppen zu beobachten waren und deutliche Entzündungszeichen infolge der Infektion darstellen.

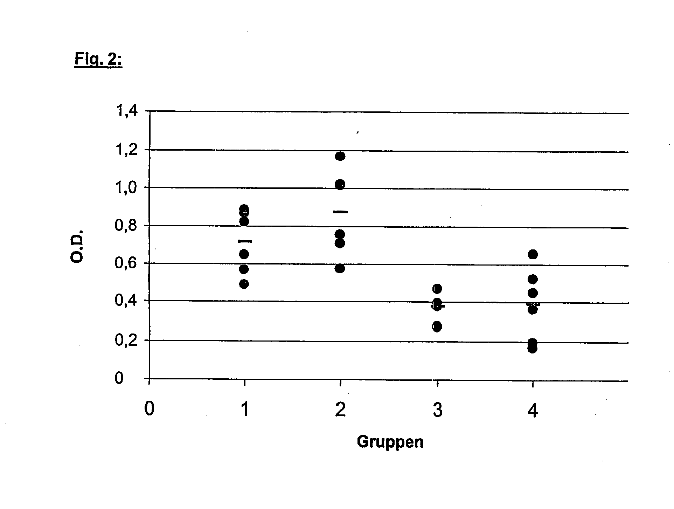

- **Fig. 2:**: Die Bestimmung des Gesamt IgG Titer im Zieltier Hund vor der Belastungsinfektion mit Leishmania infantum Promastigoten. Hierin bedeuten:
Gruppe 1: MIDGE-NLSp36 LACK
Gruppe 2: MIDGE-NLS 4-Antigen-Kombination
Gruppe 3: Plasmid 4-Antigen-Kombination
Gruppe 4: Kontroll-Gruppe: PBS

Die Punkte repräsentieren die Daten der einzelnen Hunde, während die waagerechten Balken den Mittelwert der entsprechenden Gruppe kennzeichnen. Die optische Dichte (OD) steigt mit der Konzentration der Serum-Antikörper. In Figur 2 ist ein deutlicher Unterschied zwischen den Konzentrationen der Anti-Leishmania-Antikörper der Gruppen 1 und 2 einerseits und den Gruppen 3 und 4 andererseits sichtbar. Die Hunde der Gruppe 1, die mit MIDGE-NLS-LACK geimpft wurden, und die Hunde der Gruppe 2, die die Kombination der vier Antigen-Sequenzen in MIDGE-NLS erhalten hatten, besaßen mehr Antikörper gegen Leishmania infantum als die Gruppen 3 und 4. Bemerkenswert ist, daß in Gruppe 3 keine Antikörper nachweisbar waren, obwohl die gleichen Antigen-Sequenzen wie in Gruppe 2 verwendet wurden. Dies zeigt, daß die Kombination der vier Antigensequenzen besonders in Verbindung mit MIDGE-NLS-Vektoren zur Induktion einer humoralen Immunantwort geeignet ist. Auch im Vergleich zur Gruppe 1 besaßen die Hunde der Gruppe 2 durchschnittlich mehr Antikörper. Daraus kann der Schluß gezogen werden, daß die Kombination der vier Antigene einen Vorteil gegenüber der alleinigen Verabreichung von LACK darstellt.

Im Folgenden wird näher auf die Versuchsergebnisse eingegangen; im Einzelnen:

In einem Impfversuch in Mäusen wurden verschiedene Kombinationen der eingesetzten Antigene auf ihre Wirksamkeit, einen möglichst hohen Schutz gegen Infektionen mit Leishmaniose zu erreichen, getestet. Als Parameter für eine erzielte Schutzwirkung wurde die Unterdrückung des Läsionswachstums durch die eingesetzten Vakzinekombinationen benutzt. Zur Beurteilung der Schutzwirkung wurde eine Belastungsinfektion mit Leishmania infantum Promastigoten durchgeführt. Die Beurteilung des Läsionswachstums erfolgte wöchentlich. Die Ergebnisse in Figur 1 stammen aus Woche acht nach der Belastungsinfektion. Grundsätzlich entwickelten alle Tiere, die mit einem Antigen oder einer Zweierkombination der Antigene geimpft wurden, früher Läsionen als die Tiere, die mit einer Dreier- oder Viererkombination der Antigene geimpft wurden. Ein guter Impferfolg wurde mit der Dreikombination der Antigene Kmp-11, TSA und p36 LACK erreicht. Eine vergleichbare Schutzwirkung wurde mit der Antigenkombination TSA, gp63 und Kmp-11 erreicht. Der überraschende Impferfolg wird darin sichtbar, das bei beiden erfolgreichen Dreierkombinationen (TSA, gp 63, Kmp-11 und p36 LACK, Kmp-11, gp 63) die Größe der Läsionen unterhalb der Nachweisgrenze von 0,5 Millimetern liegen und sich somit keine auswertbaren Läsionen entwickelten. Damit ist die Läsionsgröße bei den geimpften Tieren um 100% kleiner als bei der ungeimpften Kontrollgruppe.

**Tabelle 1: Zusammensetzung der Antigenkombinationen in einem Immunisierungsversuch in Mäusen.**

| Gruppe | Anzahl Tiere | Menge an DNA [in Mikrogram] |
|---|---|---|
| 1. MIDGE-NLS-p36 LACK+Kmp-11 | 9 | 50 |
| 2. MIDGE-NLS-p36 LACK+TSA | 9 | 50 |
| 3. MIDGE-NLS-p36 LACK+gp 63 | 9 | 50 |
| 4. MIDGE-NLS-Kmp11+TSA . | 9 | 50 |
| 5. MIDGE-NLS-Kmp11+gp 63 | 9 | 50 |
| 6. MIDGE-NLS-TSA+gp 63 | 9 | 50 |
| 7. MIDGE-NLS-TSA+gp 63+p36 LACK | 9 | 50 |
| 8. MIDGE-NLS TSA+gp 63+p36 LACK+Kmp-11 | 9 | 50 |
| 9. MIDGE-NLS p36 LACK | 9 | 50 |
| 10. MIDGE-NLS TSA | 9 | 50 |
| 11. MIDGE-NLS Kmp-11 | 9 | 50 |
| 12. MIDGE-NLS gp 63 | 9 | 50 |
| 13. MIDGE-NLS TSA+gp 63+Kmp-11 | 9 | 50 |
| 14. MIDGE-NLS p36 LACK+Kmp-11+gp 63 | 9 | 50 |
| 15. MIDGE-NLS p36 LACK+Kmp-11+TSA | 9 | 50 |
| 17. PBS | 9 | 50 |

Je Gruppe wurden neun weibliche Balb/c Mäuse eingesetzt. Die Tiere wurden mit 50 Mikrogramm DNA geimpft. In den Antigenkombinationen betrug die Menge an eingesetzter DNA 50 Mikrogramm pro Antigen. Nach zwei Wochen erfolgte die Zweitimmunisierung (boost). Drei Wochen nach dem Boost erfolgte die Belastungsinfektion mit 5x10⁴ Leishmania infantum Promastigoten, die sich in der stationären metazyklischen Phase befanden. Diese wurden den Tieren subkutan in die rechte Hinterpfote injiziert. Der Stand der Infektion wurde wöchentlich verfolgt. Die Größe der Läsionen wurde mit einer elektronischen Schiebelehre durch Vergleich mit der linken unbehandelten Hinterpfote bestimmt. In einem Folge-Experiment in weiblichen 4-12 Monate alten Beagle Hunden wurden ebenfalls verschiedene Impfstoffzusammensetzungen formuliert, die sich in der verwendeten Antigenkombination unterschieden. Die Negativkontrolle, Gruppe 4, erhielt nur PBS-Puffer und diente als Infektionskontrollgruppe. Die Gruppe 1 bestand aus nur einem Antigen, dem p36 LACK Antigen. Dieses Antigen hat in früheren Versuchen zu gutem Schutz geführt und sollte im Zieltier Hund überprüft werden (L. Lopez-Fuertes et al., 2002, Vaccine 21: 247-257). Die Gruppe 3 bestand aus verschiedenen Plasmiden kodierend für die Leishmania infantum Antigene: TSA, Kmp-11, gp63 und p36 LACK. Diese Gruppe dient zum Vektor- und Wirkungsvergleich mit Gruppe 2, die aus dergleichen Antigenkombination bestand, jedoch peptidgekoppelte MIDGE als Vektoren beinhaltet.

Es wurden 4 Impfgruppen mit jeweils 6 Tieren zusammengestellt, die in 15-tägigem Abstand viermal intradermal mit jeweils 200 Mikrogramm DNA pro Konstrukt immunisiert wurden (siehe Tabelle 3). Vor dem Beginn des Experimentes und nach jeder Immunisierung wurde den Tieren, neben routinemäßigen veterinärmedizinischen Untersuchungen, Blut für serologische Untersuchungen abgenommen.

Um festzustellen, welcher Art der Immunantwort durch die Impfung oder eine Infektion erzeugt wurde, sind verschiedene Methoden anwendbar. Zu den Methoden, die den Rückschluss auf eine zelluläre Immunantwort zulassen, gehört der Test auf "verzögerte Überempfindlichkeit" der auch als "Delayed Type Hypersensitivity"-Test (DTH-Test) bezeichnet wird. Durch Messung der Hautdicke an der Injektionsstelle 72 Stunden nach der Antigen-Applikation kann relativ genau bestimmt werden, ob eine verzögerte Überempfindlichkeitsreaktion und damit eine spezifische T-Zell-Reaktion auf das Antigen erfolgte. Die Ergebnisse sind nachfolgend in Tabelle 2 dargestellt:

**Tabelle 2**

| Gruppe | Hunde | DTH-Test | DTH-Test |
|---|---|---|---|
| | | vor Impfung | nach Impfung |
| MIDGE-NLSp36 LACK- | 1 | - | + |
| | 2 | - | - |
| | 3 | - | - |
| | 4 | - | + |
| | 5 | - | - |
| | 6 | - | - |
| MIDGE-NLS | 1 | - | + |
| TSA, p36, Kmp11, gp36 | 2 | - | + |
| | 3 | - | +(?) |
| | 4 | - | - |
| | 5 | - | + |
| | 6 | - | + |
| Plasmid | 1 | - | + |
| TSA, p36 LACK, Kmp11, gp36 | 2 | - | + |
| | 3 | - | - |
| | 4 | - | ++ |
| | 5 | - | - |
| | 6 | - | + |
| PBS Puffer | 1 | - | - |
| | 2 | + | - |
| | 3 | - | + |
| | 4 | - | + (?) |
| | 5 | - | - (?) |
| | 6 | - | - |

Die Tabelle 2 zeigt die Ergebnisse des Tests auf "verzögerte Überempfindlichkeit" der auch als "Delayed Type Hypersensitivity"-Test (DTH-Test) bezeichnet wird. Eine lokale Hautschwellung wird als positiver DTH-Test bewertet (in der Abbildung mit "+" oder "++" gekennzeichnet) und zeigt an, daß das betreffende Tier antigenspezifische T-Gedächtniszellen infolge der Impfung ausgebildet und somit eine zelluläre Immunantwort stattgefunden hat. Wie beschrieben ist die zelluläre Immunantwort entscheidend in der Prophylaxe und Therapie von Leishmaniose Erkrankungen. Vor Beginn des Experimentes wurde der DTH durchgeführt, um sicherzustellen, dass keines der Versuchstiere bereits eine zelluläre Immunantwort gegen L. infantum infolge einer früheren Infektion/lmpfung ausgebildet hatte. Alle Tiere, bis auf eines, reagierten im DTH Test negativ und erfüllten damit die Voraussetzung für den Versuch. Eine positive Reaktion nach Abschluß der Immunisierung bedeutet, dass die Immunisierung eine spezifische zelluläre Immunantwort gegen Leishmania infantum erzeugt hat. Das ist als Impferfolg zu werten und läßt einen besseren Schutz gegen Infektionen erwarten.

Nach der letzten Impfung wurde wieder ein DTH Test durchgeführt. Hier erwartete man, dass möglichst alle geimpften Tiere positiv reagierten. Da das zuvor positive Tier nun negativ reagierte, ist davon auszugehen, das es auch schon in dem ersten DTH Test negativ war und es sich um einen Fehler in der Interpretation des Ergebnisses handelte.

Die durch die Impfungen erzeugte Immunantwort wurde zusätzlich durch serologische Untersuchungen charakterisiert. In Fig. 2 sind die Ergebnisse des ELISA-Nachweises von spezifischen Antikörpern gegen Leishmania infantum im Serum der immunisierten Hunde dargestellt. Insgesamt waren die Antikörperkonzentrationen in den Seren der geimpften Hunde nicht sehr hoch. Dieses Ergebnis entspricht den Erwartungen, da die Immunisierung mehr auf eine zelluläre als auf eine humorale Immunantwort abzielte.

**Tabelle 3: Zusammensetzung der Impfgruppen im lmmunisierungsversuch gegen Leishmania infantum in Hunden**

| Gruppe | Verwendetes Antigen | Anzahl Tiere | Menge an DNA | Appliziertes Volumen |
|---|---|---|---|---|
| 1 | MIDGE-NLSp36 LACK | 6 | 200 µg pro Konstrukt | 500 µl pro Tier |
| 2 | MIDGE-NLS-TSA, MIDGE-NLS-Kmp-11, MIDGE-NLS-gp63, MIDGE-NLS-p36 LACK | 6 | 200 µg pro Konstrukt | 500 µl pro Tier |
| 3 | pMCV-TSA, pMVC-Kmp-11, pMCV-gp63, pMCV-p36 LACKMIDGE | 6 | 200 µg | 500 µl pro Tier |
| 4 | PBS Puffer | 6 | - | 500 µl pro Tier |

Der Erfolg der Immunisierung wird beschreibbar durch eine Belastungsreaktion der Tiere mit dem Erreger. Vier Wochen nach der letzten Immunisierung fand deshalb intravenös eine Belastungsinfektion mit 5x10⁷ Leishmania infantum Promastigoten statt. Der Grad der Infektion und damit der durch die Impfung erreichte Schutz, wird anhand klinisch-pathologischer Untersuchungen festgestellt. Zu diesen zählen die Untersuchung auf Schwellung der Lymphknoten in der Kniekehle, Gewichtsverlust, Rückbildung mit einhergehender Veränderung der Farbe der Muskeln, überschießendes Nagelwachstum, Läsionen der Haut sowie Veränderungen im Hämogramm. Das Vorhandensein und die Quantität des Erregers werden durch eine quantitative PCR-Reaktion bestimmt. Bei Vergleich der Gruppen 11 Monate nach der Belastungsinfektion in Bezug auf die Ausbildung klinischer Symptome ergibt sich folgendes Bild: Es ist ein deutlicher Unterschied zwischen Gruppe 2 und der Kontrollgruppe erkennbar. In Gruppe 2 erkrankte nur 1 Hund an Leishmaniose, während in der Kontrollgruppe 4 von 6 Hunden an Leishmaniose erkrankten. Auch in der Gruppe 1 war die Anzahl erkrankter Hunde gegenüber der Kontrollgruppe deutlich reduziert, während Gruppe 3 nur einen geringen Unterschied zur Kontrollgruppe aufwies. Der direkte Vergleich der Wirkung von MIDGE-NLS mit Plasmid kodierend für exakt die gleichen Antigene, (Gruppe 2 und Gruppe 3) zeigt, das mit MIDGE-NLS ein besserer Schutz gegen Leishmaniose erzielt wurde, als es mit Plasmid möglich war. Die Versuchsergebnisse lassen die Schlussfolgerung zu, das die Immunisierung von Hunden gegen Leishmaniose mit einer Kombination aus den vier MIDGE -NLS kodierten Antigenen, die Entwicklung klinischer Symptome der Leishmaniose in Hunden verhindern kann. Nach dem Erkenntnisstand der Anmelder ist ein vergleichbar gutes Ergebnis, wie es mit dem erfindungsgemäßen Impfstoff MIDGE-NLS kodierend für 4 Antigene erzielt wurde, bisher nur von Ramiro et al., (Vaccine 3696, 2003: 1-11) beschrieben worden. Im Unterschied zu MIDGE-NLS ist jedoch in der Studie von Ramiro et al., ein rekombinanter Vaccinia Virus (rVV) als Boostimpfstoff verwendet worden. Bei dem rekombinaten Vaccinia Virus handelt es sich um genetisch modifizierte Viren, die bekanntermaßen ein hohes Sicherheitsrisiko darstellen (Lehrman, 1999, Nature 401: 517-518).

### Ausführungsbeispiele

### Beispiel 1: Klonierung des Plasmids pMCVp36

Vom Ausgangsplasmid pSCp36 wurden 2 Fragmente mittels PCR amplifiziert:
1. PCR ca. 800 bp;
2. PCR ca. 950 bp;

Das PCR-Produkt aus der 2. PCR wurde mit Eco311 geschnitten und das kleinere Fragment (ca. 200 bp) isoliert.

Das PCR-Produkt aus der 1. PCR wurde mit Bpil geschnitten.

Das 200 bp Fragment und das geschnittene Fragment aus der 1. PCR wurden zusammenligiert und anschließend mit KpnI und SacI geschnitten und in den mit Kpnl und SacI geschnittenen pMOK-Vektor ligiert. Das entstandene Plasmid erhielt den Namen pMCVp36.

### Beispiel 2: Klonierung des Plasmids pMCVKmp-11

Das Gen wurde mittels PCR aus cDNA von Leishmania infantum amplifiziert. Das PCR-Produkt wurde nach Verdau mit Kpnl und Xhol in den ebenso geschnittenen Vektor pMCV1.4 kloniert und sequenziert. Das entstandene Plasmid wurde pMCVKpm11 genannt.

### Beispiel 3: Klonierung des Plasmids pMCVTSA

Das Gen wurde mittels PCR aus cDNA von Leishmania infantum amplifiziert und durch einfügen eines Basenaustausches, ohne die Aminosäuresequenz zu verändern, eine Eco31I-Erkennungssequenz entfernt. Das abschließende PCR-Produkt wurde nach Verdau mit Kpnl und XhoI in den ebenso geschnittenen Vektor pMCV1.4 kloniert und sequenziert. Das entstandene Plasmid wurde pMCVTSA genannt.

### Beispiel 4: Klonierungsstrategie für die Codon-Usage Optimierung von gp63

Die Oligonukleotide wurden mit einer Länge zwischen 18 und 28 Basen bestellt (MWG Biotech). Zweimal 90 Oligonukleotide, die jeweils den Vorwärts- und Rückwärtsstrang abbilden, wurden durch Annealing und Ligation miteinander verbunden. Die annealten Oligonukleotide erzeugten jeweils zwei 4-Basen Überhänge. Es wurde darauf geachtet, dass die Überhänge nur einmal vorkamen und nicht palindromisch waren. Die Oligonukleotide wurden annealt, indem die beiden einzelnen Oligonukleotide (Strang und Gegenstrang) mit Kinasepuffer versetzt auf ca. 80°C erhitzt wurden und dann langsam auf Raumtemperatur abgekühlt wurden. Danach wurde ATP und Polynukleotidkinase (PNK) zugegeben und die Oligonukleotide für eine Stunde phosphoryliert. Anschließend wurden im ersten Schritt jeweils benachbarte Oligonukleotide zusammengegeben und ligiert (Oligonukleotid 1+2, Oligonukleotid 3+4). Nach 1 h Ligation wurde ein Aliquot vom Ligationsansatz der Oligonukleotide 1+2 und ein Aliquot vom Ligationsansatz 3+4 zusammengegeben. Dies wurde mit den verschiedenen Ligationsansätzen bis zu einer Länge des Ligationsproduktes von etwa 300bp durchgeführt. Ein Aliquot des jeweils letzten Ligationsschrittes wurde als Template in eine PCR mit flankierenden Primern eingesetzt. Das PCR-Produkt wurde in den TOPO-TA-Cloning Vektor (Invitrogen) zwischenkloniert und sequenziert. Dies erfolgte mit insgesamt sechs Fragmenten des kompletten Gens. Die sechs einzelnen Fragmente wurden aus dem zwischenklonierten Plasmid mit Eco311 ausgeschnitten und miteinander ligiert. Das gesamte Ligationsprodukt richtiger Länge wurde in einer abschließenden PCR amplifiziert, mit KpnI und Sac I verdaut, und in den ebenso geschnittenen Vektor pMCV1.4 nach Gelextraktion kloniert. Anschließend wurde die Sequenz durch Sequenzierung kontrolliert. Das entstandene Plasmid wurde pMCVgp63 genannt.

Primer für die 6 zusammengesetzten Fragmente:
Fragment 1:
Fragment 2:
Fragment 3:
Fragment 4:
Fragment 5:
Fragment 6:
gesamte Sequenz:

### Beispiel 5: Kopplung der NLS Sequenz an Oligonukleotide

Die NLS Kopplung wurden wie folgt vorgenommen: das NLS Peptid mit der Sequenz PKKKRKV wurde in zwei Schritten an die ODN's gekoppelt. Zuerst wurde das modifizierte Oligonukleotid 5'-PH-dGGG AGT CCA GT xT TTC TGG AC (wobei xT für aminomodifizierte Thyminbase mit C₂ - Amminolinker steht, = ODN 1) (0,1mM) mit sulfo-KMUS (5mM) in PBS bei Raumtemperatur (RT) aktiviert. Nach 120 min. wurde die Reaktion mit 50 mM Tris-(Hydroxymethyl)-Aminomethane gestoppt und das aktivierte ODN nach Ethanolpräzipation (300mM NaOAc pH 5.2, 5.5 mM MgCl₂, 70 % Ethanol), Zentrifugation und einmaligem Waschen mit 70% Ethanol erhalten. Das so erhaltene ODN (0,1mM) wurde in PBS gelöst und reagierte mit dem Peptid (0,2mM) für eine Stunde bei Raumtemperatur. Die Reaktion wurde durch Gelelektrophorese (3%) und Ethidiumbromidfärbung überprüft. Das entstandene NLS gekoppelte ODN wurde durch HPLC aufgereinigt und zur Synthese der MIDGE-NLS Antigen Konstrukte verwendet.

### Beispiel 6: Herstellung der MIDGE-NLSp36

MIDGE sind lineare, kovalent geschlossene Expressionskassetten, die lediglich aus dem CMV Promotor, einem Intron, der entsprechenden Gensequenz und einer Polyadenylierungssequenz bestehen (vgl. EP 0 941 318 B1). Die Konstrukte wurden wie folgt erhalten: das unter Beispiel 1.1 beschriebene Plasmid pMCVp36 wurde mit Eco 311 vollständig verdaut. Die Ligation mit 5' phosphorylierten haarnadelförmigen Oligodesoxynukleotiden (ODN) 5'-PH-GGG AGT CCA GT XT TTC TGG AC (= ODN 1) und 5'-AGG GGT CCA GTT TTC TGG AC-3'(= ODN 2) durch T4 DNA Ligase in Anwesenheit von Eco 311 wurde durch Erhitzen auf 70°C gestoppt. Das resultierende Gemisch wurde konzentriert und mit Eco 311 und T7 DNA Ligase in Abwesenheit von Deoxyribonukleotid-Triphosphaten behandelt. Die Aufreinigung erfolgte durch Anionenaustauschchromatographie.

Die Herstellung von MIDGE-NLS-TSA, -Kmp-11 und gp63 erfolgte analog.

### Beispiel 7: DHT Test

Test auf "verzögerte Überempfindlichkeit" der auch als "Delayed Type Hypersensitivity"-Test (DTH-Test) bezeichnet wird. Bei diesem Test wird dem geimpften Tier eine sehr kleine Menge Antigen in die obersten Hautschichten verabreicht. Zuvor wird die Hautdicke an der Applikationsstelle genau gemessen. Anschließend beobachtet man, ob sich die Haut an der Injektionsstelle entzündlich verändert. Charakteristisch für eine durch sensibilisierte T-Zellen hervorgerufene Immunreaktion auf das Antigen ist eine Entzündungsreaktion, die erst nach 48 bis 72 Stunden nachweisbar ist. Die klinischen Symptome, wie lokale Hautschwellung, Rötung und eventuell Schmerzhaftigkeit, können auf die Wirkung von Zytokinen zurückgeführt werden.. Eine lokale Hautschwellung wird als positiver DTH-Test bewertet (in der Tabelle 1 mit "+" oder "++" gekennzeichnet) und zeigt an, daß das betreffende Tier antigen-spezifische T-Gedächtniszellen infolge der Impfung ausgebildet hat.

### Beispiel 8: Bestimmung Gesamt-Antikörper nach Immunisierung

In Fig. 2 sind die Ergebnisse des ELISA-Nachweises von spezifischen Antikörpern gegen Leishmania infantum im Serum der immunisierten Hunde dargestellt. Vor der Belastungsinfektion wurden von allen Hunden die Seren gewonnen. Die Bestimmung des Gesamt IgG Antikörpertiter erfolgte mittels ELISA, wobei die Absorption in OD (Optische Dichte) bei einer Wellenlänge von λ = 406 nm bestimmt wurde.

## Patentansprüche

1. Verwendung eines DNA-Expressionskonstruktes zur Herstellung eines Arzneimittels zur Immunisierung gegen Leishmaniose-Infektionen, wobei besagtes DNA-Expressionskonstrukt ein oder mehrere kodierende Nukleinsäuresequenzen enthält, welche zur Expression der *Leishmania infantum* Antigene *thiol-specific antioxidant protein Gen* (TSA), Glykoprotein gp63 und *kinetoplastid membrane protein 11* (Kmp-11) führen.

2. Verwendung von zwei oder drei DNA-Expressionskonstrukten zur Herstellung eines Arzneimittels zur Immunisierung gegen Leishmaniose-Infektionen, wobei besagte DNA-Expressionskonstrukte jeweils ein oder mehrere kodierende Nukleinsäuresequenzen enthalten, die gemeinsam zur Expression der *Leishmania infantum* Antigene *thiol-specific antioxidant protein Gen* (TSA), Glykoprotein gp63 und *kinetoplastid membrane protein 11* (Kmp-11) führen.

3. Verwendung nach Anspruch 1 oder 2, wobei wenigstens zwei der Leishmania infantum Antigene als Fusionsprotein exprimiert werden.

4. Verwendung nach Anspruch 3, wobei das Fusionsprotein das Expressionsprodukt aus *thiol-specific antioxidant protein Gen* (TSA) und *kinetoplastid membrane protein 11* Gen (Kmp-11) ist.

5. Verwendung nach Anspruch 3 und 1, wobei das Fusionsprotein das Expressionsprodukt aus *thiol-specific antioxidant protein Gen* (TSA), Glykoprotein gp63 Gen und *kinetoplastid membrane protein 11* Gen (Kmp-11) ist.

6. Verwendung nach wenigstens einem der Ansprüche 1 bis 5, wobei zusätzlich ein DNA-Expressionskonstrukt zur Expression des Leishmania Antigen p36 LACK enthalten ist.

7. Verwendung nach wenigstens einem der Ansprüche 1 bis 6, wobei die kodierenden Polynukleotidsequenzen als Expressionskonstrukte vorliegen, die aus kovalent geschlossenen linearen Desoxyribonukleinsäuremolekülen bestehen, welche einen linearen Doppelstrangbereich aufweisen, wobei die doppelstrangbildenden Einzelstränge durch kurze einzelsträngige Schlaufen aus Desoxyribonukleinsäurenukleotiden verknüpft sind, wobei die doppelstrangbildenden Einzelstränge nur aus der kodierenden Sequenz unter Kontrolle eines im zu impfenden Tier operablen Promotors und einer Terminatorsequenz bestehen.

8. Verwendung nach einem der Ansprüche 1 bis 6, wobei die kodierenden Polynukleotidsequenzen in einem zirkulär doppelsträngigen Expressionsvektor vorliegen.

9. Verwendung nach wenigstens einem der vorhergehenden Ansprüche, wobei das DNA-Expressionskonstrukt zur Steigerung der Transfektionseffizienz mit einem oder mehreren Oligopeptiden kovalent verknüpft ist.

10. Verwendung nach wenigstens einem der vorhergehenden Ansprüche, wobei die DNA-Expressionskonstrukte jeweils mit einem Oligopeptid aus 3 bis 30 Aminosäuren konjugiert sind, wobei das Oligopeptid zur Hälfte aus basischen Aminosäuren aus der Gruppe Arginin und Lysin besteht.

11. Verwendung nach Anspruch 10, wobei die konjugierten Oligopeptide die Aminosäuresequenz YGRKKRRQRRR aufweisen.

12. Verwendung nach wenigstens einem der vorhergehenden Ansprüche, wobei die linear-kovalent geschlossenen DNA-Expressionskonstrukte mit einem die Kernlokalisationssequenz (NLS) des large T-Antigens von SV40 enthaltenden Peptid mit der Aminosäuresequenz PKKKRKV modifiziert sind.

13. Verwendung eines DNA-Expressionskonstruktes zur Herstellung eines Impfstoffes zur Behandlung von Leishmaniose-Infektionskrankheiten, wobei besagtes DNA-Expressionskonstrukt ein oder mehrere kodierende Nukleinsäuresequenzen enthält, welche zur Expression der *Leishmania infantum* Antigene *thiol-specific antioxidant protein Gen* (TSA), Glykoprotein gp63 und *kinetoplastid membrane protein 11* (Kmp-11) führen.

14. Verwendung von zwei oder drei DNA-Expressionskonstrukten zur Herstellung eines Impfstoffes zur Behandlung von Leishmaniose-Infektionskrankheiten, wobei besagte DNA-Expressionskonstrukte jeweils ein oder mehrere kodierende Nukleinsäuresequenzen enthalten, die gemeinsam zur Expression der *Leishmania infantum* Antigene *thiol-specific antioxidant protein Gen* (TSA), Glykoprotein gp63 und *kinetoplastid membrane protein 11* (Kmp-11) führen.

15. DNA-Expressionskonstrukt, enthaltend ein oder mehrere kodierende Nukleinsäuresequenzen, welche zur Expression der *Leishmania infantum* Antigene *thiol-specific antioxidant protein Gen* (TSA), Glykoprotein gp63 und *kinetoplastid membrane protein 11* (Kmp-11) führen.

16. DNA-Expressionskonstrukt nach Anspruch 15, wobei die Genprodukte als ein singuläres Fusionsprotein exprimiert werden.

17. DNA-Expressionskonstrukt, enthaltend ein oder mehrere kodierende Nukleinsäuresequenzen, welche zur Expression der *Leishmania infantum* Antigene *thiol-specific antioxidant protein Gen* (TSA) und *kinetoplastid membrane protein 11* (Kmp-11) führen.

18. DNA-Expressionskonstrukt nach Anspruch 17, wobei die Genprodukte als ein singuläres Fusionsprotein exprimiert werden.

19. DNA-Expressionskonstrukt nach wenigstens einem der Ansprüche 15-18, wobei das Expressionskonstrukt als kovalent geschlossenes lineares Desoxyribonukleinsäuremoleküle vorliegt, welches einen linearen Doppelstrangbereich aufweist, wobei die doppelstrangbildenden Einzelstränge durch kurze einzelsträngige Schlaufen aus Desoxyribonukleinsäurenukleotiden verknüpft sind, wobei die doppelstrangbildenden Einzelstränge nur aus der kodierenden Sequenz unter Kontrolle eines im zu impfenden Tier operablen Promotors und einer Terminatorsequenz bestehen.

20. DNA-Expressionskonstrukt nach wenigstens einem der Ansprüche 15-18, wobei die kodierenden Polynukleotidsequenzen in einem zirkulär doppelsträngigen Expressionsvektor vorliegen.

21. DNA-Expressionskonstrukt nach wenigstens einem der Ansprüche 15 - 20, wobei das DNA-Expressionskonstrukt zur Steigerung der Transfektionseffizienz mit einem oder mehreren Oligopeptiden kovalent verknüpft ist.

22. DNA-Expressionskonstrukt nach Anspruch 21, wobei das DNA-Expressionskonstrukt mit einem Oligopeptid aus 3 bis 30 Aminosäuren konjugiert ist, wobei das Oligopeptid zur Hälfte aus basischen Aminosäuren aus der Gruppe Arginin und Lysin besteht.

23. DNA-Expressionskonstrukt nach Anspruch 22, wobei das konjugierte Oligopeptid die Aminosäuresequenz YGRKKRRQRRR aufweist.

24. DNA-Expressionskonstrukt nach Anspruch 21, wobei das linear-kovalent geschlossenen DNA-Expressionskonstrukte mit einem die Kernlokalisationssequenz (NLS) des large T-Antigens von SV40 enthaltenden Peptid mit der Aminosäuresequenz PKKKRKV modifiziert ist.

25. DNA-Expressionskonstrukt nach Anspruch 15 oder 16, wobei die Sequenz für gp63 kodon-optimiert ist.

26. DNA-Expressionskonstrukt nach Anspruch 25, enthaltend die Sequenz Seq.ID 5.

27. Vakzine zur Behandlung von Leishmaniose-Infektionskrankheiten, enthaltend ein DNA-Expressionskonstrukt nach einem oder mehreren der Ansprüche 15 bis 26.

28. Vakzine nach Anspruch 27, welche zusätzlich Adjuvantien und/oder immunstimulatorische Nukleinsäuresequenzen mit einem oder mehreren CpG-Motiven enthält.

29. Vakzine nach Anspruch 28, wobei die immunstimulatorische Nukleinsäuresequenzen als zirkulärer Strang Desoxyribonukleinsäure mit einer teilweise zueinander komplementären, antiparallelen Basensequenz vorliegen.

## Claims

1. Method for using a DNA expression construct for the production of a drug for the immunisation against leishmaniasis infections, wherein said DNA expression construct contains one ore more coding nucleic acid sequences, which lead to the expression of the *Leishmania infantum* antigens *thiol-specific antioxidant protein gene* (TSA), glycoprotein gp63 and *kinetoplastid membrane protein 11* (Kmp-11).

2. Method for using two or three DNA expression constructs for the production of a drug for the immunisation against leishmaniasis infections, wherein said DNA expression constructs respectively contain either one ore more coding nucleic acid sequences, that collectively lead to the expression of the *Leishmania infantum* antigens *thiol-specific antioxidant protein gene* (TSA), glycoprotein gp63 and *kinetoplastid membrane protein 11* (Kmp-11).

3. Method according to claim 1 or 2, wherein at least two of the *Leishmania infantum* antigens are expressed as fusion proteins.

4. Method according to claim 3, wherein the fusion protein is the expression product of *thiol-specific antioxidant protein gene* (TSA) and *kinetoplastid membrane protein 11 gene* (Kmp-11).

5. Method according to claim 3 and 1, wherein the fusion protein is the expression product of *thiol-specific antioxidant protein gene* (TSA), glycoprotein gp63 gene and *kinetoplastid membrane protein 11 gene* (Kmp-11).

6. Method according to at least one of claims 1 to 5, wherein additionally a DNA expression construct for the expression of the *Leishmania* antigen p36 LACK is contained.

7. Method according to at least one of claims 1 to 6, wherein the coding polynucleotide sequences are expression constructs, that consist of covalently closed linear deoxyribonucleic acid molecules, which have a linear double stranded region, wherein the double strand forming single strands are linked by short single stranded loops of deoxyribonucleic acid nucleotides, wherein the double strand forming single strands consist only of the coding sequence under control of a promoter that is operable in the animal that is to be vaccinated and a terminator sequence.

8. Method according to at least one of claims 1 to 6, wherein the coding polynucleotide sequences are present as a circular double stranded expression vector.

9. Method according to at least one of the previous claims, wherein the DNA expression construct is covalently linked to one or more oligopeptides to increase transfection efficiency.

10. Method according to at least one of the previous claims, wherein each of the DNA expression constructs are linked to an oligopeptide of 3 to 30 amino acids, wherein the oligopeptide consists half of basic amino acids selected from the groups arginine and lysine.

11. Method according to claim 10, wherein the linked oligopeptides have the amino acid sequence YGRKKRRQRRR.

12. Method according to at least one of the previous claims, wherein the linear covalently closed DNA expression constructs are modified with a peptide containing the nuclear localisation sequence (NLS) of the large T-antigen from SV40 with the amino acid sequence PKKKRKV.

13. Method for using a DNA expression construct for the production of a vaccine for the treatment of leishmaniasis infectious diseases, wherein said DNA expression construct contains one ore more coding nucleic acid sequences, that lead to the expression of the *Leishmania infantum* antigens *thiol-specific antioxidant protein gene* (TSA), glycoprotein gp63 and *kinetoplastid membrane protein 11* (Kmp-11).

14. Method for using two or three DNA expression constructs for the production of a vaccine for the treatment of leishmaniasis infectious diseases, wherein said DNA expression constructs respectively contain either one ore more coding nucleic acid sequences, that collectively lead to the expression of the *Leishmania infantum* antigens *thiol-specific antioxidant protein gene* (TSA), glycoprotein gp63 and *kinetoplastid membrane protein 11* (Kmp-11).

15. DNA expression construct, containing one or more coding nucleic acid sequences, which lead to the expression of the *Leishmania infantum* antigens *thiol-specific antioxidant protein gene* (TSA), glycoprotein gp63 and *kinetoplastid membrane protein 11* (Kmp-11).

16. DNA expression construct according to claim 15, wherein the gene products are expressed as a single fusion protein.

17. DNA expression construct, containing one or more coding nucleic acid sequences, which lead to the expression of the *Leishmania infantum* antigens *thiol-specific antioxidant protein gene* (TSA) and *kinetoplastid membrane protein* 11 (Kmp-11).

18. DNA expression construct according to claim 17, wherein the gene products are expressed as a single fusion protein.

19. DNA expression construct according to at least one of claims 15 to 18, wherein the expression construct is a covalently closed linear deoxyribonucleic acid molecule, which has a linear double stranded region, wherein the double strand forming single strands are linked by short single stranded loops of deoxyribonucleic acid nucleotides, wherein the double strand forming single strands consist only of the coding sequence under control of a promoter that is operable in the animal that is to be vaccinated and a terminator sequence.

20. DNA expression construct according to at least one of claims 15 to 18, wherein the coding polynucleotide sequences are present as a circular double stranded expression vector.

21. DNA expression construct according to at least one of claims 15 to 20, wherein the DNA expression construct is covalently linked to one or more oligopeptides to increase transfection efficiency.

22. DNA expression construct according to claim 21, wherein the DNA expression construct is linked to an oligopeptide of 3 to 30 amino acids, wherein the oligopeptide consists half of basic amino acids selected from the groups arginine and lysine.

23. DNA expression construct according to claim 22, wherein the linked oligopeptide has the amino acid sequence YGRKKRRQRRR.

24. DNA expression construct according to claim 21, wherein the linear covalently closed DNA expression construct is modified with a peptide containing the nuclear localisation sequence (NLS) of the large T-antigen from SV40 with the amino acid sequence PKKKRKV.

25. DNA expression construct according to claim 15 ore 16, wherein the sequence for gp63 is codon optimised.

26. DNA expression construct according to claim 25, containing the sequence Seq.ID 5.

27. Vaccine for the treatment of leishmaniasis infectious diseases, containing a DNA expression construct according to one ore more of claims 15 to 26.

28. Vaccine according to claim 27 that additionally contains adjuvants and/or immune stimulatory nucleic acid sequences with one or more CpG-motifs.

29. Vaccine according to claim 28, wherein the immune stimulatory nucleic acid sequences are present as circular strand of deoxyribonucleic acid, with a partially complementary antiparallel base sequence.

## Revendications

1. Utilisation d'un produit de recombinaison d'expression d'ADN pour fabriquer un médicament destiné à l'immunisation contre des infections de type leishmaniose, dans laquelle ledit produit de recombinaison d'expression d'ADN contient une ou plusieurs séquences d'acides nucléiques codantes, qui mènent à l'expression des antigènes de *Leishmania infantum* TSA (*gène de protéine antioxydante thiol-spécifique),* glycoprotéine gp63 et kmp-11 *(protéine 11 de la membrane cinétoplaste).*

2. Utilisation de deux ou de trois produits de recombinaison d'expression d'ADN pour fabriquer un médicament destiné à l'immunisation contre des infections de type leishmaniose, dans laquelle lesdits produits de recombinaison d'expression d'ADN contiennent respectivement une ou plusieurs séquences d'acides nucléiques codantes, qui mènent conjointement à l'expression des antigènes de *Leishmania infantum* TSA *(gène de protéine antioxydante thiol-spécifique),* glycoprotéine gp63 et kmp-11 *(protéine 11 de la membrane cinétoplaste).*

3. Utilisation selon la revendication 1 ou 2, dans laquelle au moins deux des antigènes de *Leishmania infantum* sont exprimés en tant que protéine de fusion.

4. Utilisation selon la revendication 3, dans laquelle la protéine de fusion est le produit d'expression du gène TSA (*protéine antioxydante thiol-spécifique*) et du gène kmp-11 (*protéine 11 de la membrane cinétoplaste*).

5. Utilisation selon la revendication 3 et 1, dans laquelle la protéine de fusion est le produit d'expression du gène TSA (*protéine antioxydante thiol-spécifique),* de la glycoprotéine gp63 et de kmp-11 *(protéine 11 de la membrane cinétoplaste).*

6. Utilisation selon au moins l'une des revendications 1 à 5, dans laquelle est en outre contenu un produit de recombinaison d'expression d'ADN permettant l'expression de l'antigène de leishmania p36 LACK.

7. Utilisation selon au moins l'une des revendications 1 à 6, dans laquelle les séquences codantes de polynucléotides se présentent en tant que produits de recombinaison d'expression qui se composent de molécules d'acide désoxyribonucléique linéaires et fermées de façon covalente présentant une zone bicaténaire linéaire, les simples brins qui forment le double brin étant reliés par de courtes boucles monocaténaires composées de nucléotides d'acide désoxyribonucléique, les simples brins qui forment le double brin se composant uniquement de la séquence codante sous le contrôle d'un promoteur opérable dans l'animal à inoculer et d'une séquence terminatrice.

8. Utilisation selon l'une des revendications 1 à 6, dans laquelle les séquences de polynucléotides codantes se présentent dans un vecteur d'expression bicaténaire circulaire.

9. Utilisation selon au moins l'une des revendications précédentes, dans laquelle le produit de recombinaison d'expression d'ADN est relié de façon covalente à un ou plusieurs oligopeptides pour augmenter l'efficacité de la transfection.

10. Utilisation selon au moins l'une des revendications précédentes, dans laquelle les produits de recombinaison d'expression d'ADN sont respectivement conjugués à un oligopeptide composé de 3 à 30 acides aminés, l'oligopeptide se composant pour moitié d'acides aminés basiques issus du groupe de l'arginine et de la lysine.

11. Utilisation selon la revendication 10, dans laquelle les oligopeptides conjugués présentent la séquence d'acides aminés YGRKKRRQRRR.

12. Utilisation selon au moins l'une des revendications précédentes, dans laquelle les produits de recombinaison d'expression d'ADN linéaires et fermés de façon covalente sont modifiés par un peptide de séquence d'acides aminés PKKKRKV contenant la séquence de localisation du noyau (NLS) de l'antigène Grand T du SV40.

13. Utilisation d'un produit de recombinaison d'expression d'ADN pour fabriquer un vaccin destiné au traitement de maladies infectieuses de type leishmaniose, dans laquelle ledit produit de recombinaison d'expression d'ADN contient une ou plusieurs séquences d'acides nucléiques codantes, qui mènent à l'expression des antigènes de *Leishmania infantum* TSA *(gène de protéine antioxydante thiol-spécifique),* glycoprotéine gp63 et kmp-11 *(protéine 11 de la membrane cinétoplaste).*

14. Utilisation de deux ou de trois produits de recombinaison d'expression d'ADN pour fabriquer un vaccin destiné au traitement de maladies infectieuses de type leishmaniose, dans laquelle lesdits produits de recombinaison d'expression d'ADN contiennent respectivement une ou plusieurs séquences d'acides nucléiques codantes, qui mènent conjointement à l'expression des antigènes de *Leishmania infantum* TSA *(gène de protéine antioxydante thiol-spécifique),* glycoprotéine gp63 et kmp-11 *(protéine 11 de la membrane cinétoplaste).*

15. Produit de recombinaison d'expression d'ADN contenant une ou plusieurs séquences d'acides nucléiques codantes qui mènent à l'expression des antigènes de *Leishmania infantum* TSA *(gène de protéine antioxydante thiol-spécifique),* glycoprotéine gp63 et kmp-11 *(protéine 11 de la membrane cinétoplaste).*

16. Produit de recombinaison d'expression d'ADN selon la revendication 15, dans lequel les produits génétiques sont exprimés en tant que protéine de fusion unique ou singulière.

17. Produit de recombinaison d'expression d'ADN contenant une ou plusieurs séquences d'acides nucléiques codantes qui mènent à l'expression des antigènes de *Leishmania infantum* TSA *(gène de protéine antioxydante thiol-spécifique)* et kmp-11 (gène de *protéine 11 de la membrane cinétoplaste).*

18. Produit de recombinaison d'expression d'ADN selon la revendication 17, dans lequel les produits génétiques sont exprimés en tant que protéine de fusion unique ou singulière.

19. Produit de recombinaison d'expression d'ADN selon au moins l'une des revendications 15 à 18, dans lequel le produit de recombinaison d'expression se présente sous la forme d'une molécule d'acide désoxyribonucléique linéaire et fermée de façon covalente, qui présente une zone bicaténaire linéaire, les simples brins qui forment le double brin étant reliés par de courtes boucles monocaténaires composées de nucléotides d'acide désoxyribonucléique, les simples brins qui forment le double brin se composant uniquement de la séquence codante sous le contrôle d'un promoteur opérable dans l'animal à inoculer et d'une séquence terminatrice.

20. Produit de recombinaison d'expression d'ADN selon au moins l'une des revendications 15 à 18, dans lequel les séquences de polynucléotides codantes se présentent dans un vecteur d'expression bicaténaire circulaire.

21. Produit de recombinaison d'expression d'ADN selon au moins l'une des revendications 15 à 20, dans lequel le produit de recombinaison d'expression d'ADN est relié de façon covalente à un ou plusieurs oligopeptides pour augmenter l'efficacité de la transfection.

22. Produit de recombinaison d'expression d'ADN selon la revendication 21, dans lequel le produit de recombinaison d'expression d'ADN est conjugué à un oligopeptide composé de 3 à 30 acides aminés, l'oligopeptide se composant pour moitié d'acides aminés basiques issus du groupe de l'arginine et de la lysine.

23. Produit de recombinaison d'expression d'ADN selon la revendication 22, dans lequel l'oligopeptide conjugué présente la séquence d'acides aminés YGRKKRRQRRR.

24. Produit de recombinaison d'expression d'ADN selon la revendication 21, dans lequel les produits de recombinaison d'expression d'ADN linéaires et fermés de façon covalente sont modifiés par un peptide de séquence d'acides aminés PKKKRKV contenant la séquence de localisation du noyau (NLS) de l'antigène Grand T du SV40.

25. Produit de recombinaison d'expression d'ADN selon la revendication 15 ou 16, dans lequel la séquence est optimisée par ou au niveau du codon pour la gp63.

26. Produit de recombinaison d'expression d'ADN selon la revendication 25, contenant la séquence ID. SEQ. 5.

27. Vaccins destinés à traiter des maladies infectieuses de type leishmaniose, contenant un produit de recombinaison d'expression d'ADN selon une ou plusieurs des revendications 15 à 26.

28. Vaccins selon la revendication 27, qui contiennent en outre des adjuvants et/ou des séquences d'acides nucléiques immunostimulantes ayant un ou plusieurs motifs CpG.

29. Vaccins selon la revendication 28, dans lesquels les séquences d'acides nucléiques immunostimulantes se présentent en tant qu'acide désoxyribonucléique à brin circulaire présentant une séquence de bases antiparallèles partiellement complémentaires entre elles.
